# EUROPEAN PATENT APPLICATION

(11) **EP 3 703 072 A1**
(43) Date of publication of application: **02.09.2020**
(21) Application number: 19382154.3
(22) Date of filing: 01.03.2019
(51) Int. Cl.: G16H 40/20, G16H 10/40, G06K 7/10

(54) **METHOD AND SYSTEM FOR MONITORING AND PREVENTING ERRORS IN THE USE OF BIOLOGICAL AND PHARMACEUTICAL PRODUCTS**

(71) Applicant: AT-Biotech Traceability Information Systems, SL, 28009 Madrid (ES)
(72) Inventor: AGEA MERINO, Aida, 28014 Madrid (ES); CUMBRERA TIRADO, Boris, 28051 Madrid (ES)
(74) Representative: Juncosa Miró, Jaime

(57) **Abstract**

A method and system for monitoring and preventing errors in the use of biological and pharmaceutical products are disclosed. The method comprises attaching a radio frequency sensor to a product to be used; wirelessly reading a unique identifier of the sensor and information about the product via a radio frequency reader, providing radio frequency sensor data; c) checking, by the reader, whether the provided radio frequency sensor data fulfills a given condition and/or is allowed for a given action; generating, by the reader, a certificate of compliance validating that the product is compliant with said given condition and/or action; transmitting, by the reader, the generated certificate of compliance to the sensor and to computing nodes participating in a certification system based on a blockchain protocol; and generating, by the reader, a traceability record that indicates all the events to which the product has been exposed during said use in view of the readings.

## Description

### Technical Field

The present invention is directed, in general, to methods for handling and/or processing biological and/or pharmaceutical products. In particular, the invention relates to a method and system for monitoring and preventing errors in the use of biological and pharmaceutical products.

By use of the biological and pharmaceutical products it should be understood the obtention, acquisition, manufacturing, distribution and/or dispensing processes to which the products are involved.

By biological and pharmaceutical products it should be understood any product/item such as blood, blood components, tissues, organs, and pharmaceutical products obtained from a biologic origin after a manipulation or transformation process.

### Background of the Invention

The operations to obtain and use biological and pharmaceutical products are considered of public interest in most of the countries in the world. There are local, national and international institutions dedicated to foster best practices and recommendations and some of them are mandatory. Some of these institutions are the World Health Organization, the International Society of Blood Transfusion, the European Blood Association, the Red Cross, the US FDA and many others.

The processes to pick and treat the mentioned products to be reused or transformed into new products are complex, composed of many steps. There are people involved in different locations, some physical parameters must be maintained within a certain range (temperature and pressure among others), various types of machines are used and strict conditions of cleanness and prevention of contamination must be observed.

A holistic approach to the process has been covered by different rules, directives, protocols and recommendations given by national and international organizations and improved by the suggestions of many professionals in the field along the years. The existence of software applications to manage a huge amount of information and the new machines built to reduce human manipulation has been useful to increase control and to reduce errors and mistakes. Nevertheless, the fragmented responsibility of all interveners, the gap between the information systems and the real things and the complex nature of the process itself are responsible of the prevalence of some important errors with impact on patient safety and with severe economic consequences.

Currently, the universal standard to trace and prevent errors is the barcode technology in combination with Information Systems to support the processes. Nevertheless, the cost of updating the information related to every single unit, when a transformation or movement affects it, is too high. Accordingly, the control is applied at group level, at certain points along the obtention, manufacturing or distribution cycle or by statistical means, extrapolating the results to have a picture of the whole and despite this; it is not enough to completely remove errors.

Chinese patent application CN 107392625A discloses a block-chain-based distributed pharmaceutical product source tracing method and apparatus. According to this method, a pharmaceutical manufacturer manufactures pharmaceutical products; when the pharmaceutical products are going to be transported out of a factory, tags are pasted to the pharmaceutical products. Before the pharmaceutical products enter a circulation link, the pharmaceutical manufacturer scans the tags to write unique marking information in the tags into block chains, and the block chains synchronizes the stored unique marking information to other nodes based on a consensus algorithm. Thus, in this invention, the distributed pharmaceutical product source tracing is realized by block chains, and so occurrence of the conflict between the patients and doctors is reduced.

US patent application US 2018211718 discloses methods for monitoring, collection, analysis, use and tracking of personal data, biometric data, medical data, transaction data, electronic payment data, location data and other data to develop a profile for one or more end user, pet, livestock, dairy cows, cattle or other animal using radio and other frequency tags and relaying data from EMFID tag interactions to a database that can be accessed by members of a network, and further including the use of unmanned surveillance vehicles, satellites or hand-held devices for monitoring, collection, and/or analysis of EMFID data.

However, more strategies for monitoring biological and pharmaceutical products are needed in order to prevent errors during the use of said products.

### Description of the Invention

To that end the present invention proposes, according to one aspect, a method for monitoring and preventing errors in the use of biological and pharmaceutical products. In this context, error means at least any action that breaks the link between the different elements composing the product, the product and its antecessors or predecessors of the product and the information indispensable for its safe use, among other; any action to potentially damage or turn unsafe the product; or any action against the law, rules, protocols, contracts, procedures and recommendations established by the authorities of the field to achieve the level of quality required.

Unlike the proposals known in the field, the proposed method comprises: a) attaching a radio frequency sensor, such as a RFID sensor, to a biological or pharmaceutical product to be used, the radio frequency sensor having associated thereto a unique identifier and including information about the biological or pharmaceutical product; b) wirelessly reading, at least at a given period of time during said use, the unique identifier of the radio frequency sensor and information about the biological or pharmaceutical product via a radio frequency reader such as a RFID reader, providing radio frequency sensor data; c) checking, by the radio frequency reader, whether the provided radio frequency sensor data fulfills a given condition and/or is allowed for a given action by comparing the radio frequency sensor data with control specifications of the biological or pharmaceutical product, the radio frequency reader further confirming that the radio frequency sensor data fulfills said given condition and/or is allowed for said given action;d) generating, by the radio frequency reader, a certificate of compliance that validates that the biological or pharmaceutical product is compliant with said given condition and/or action, the certificate of compliance including a chain of bits coding a given data, the given data at least including an identifier of the certificate of compliance, the unique identifier of the radio frequency sensor and control specifications of the certificate of compliance; e) transmitting, by the radio frequency reader, the generated certificate of compliance to the radio frequency sensor and to a plurality of computing nodes participating in a certification system based on a blockchain protocol, the radio frequency sensor and each of the plurality of computing nodes further storing the certificate of compliance in a memory thereof; and f) generating, by the radio frequency reader, a traceability record that indicates all the events to which the biological or pharmaceutical product has been exposed during said use in view of the readings of the radio frequency sensor by the radio frequency reader.

According to the invention, the control specifications of the biological or pharmaceutical product include the protocols, contracts or conditions to fulfil by the biological or pharmaceutical product and/or a list of actions allowed or denied for the biological or pharmaceutical product. The control specifications can be stored in a remote database or alternatively can be stored, at least in part, in a memory of the radio frequency reader.

Likewise, the traceability record includes the unique identifier of the radio frequency sensor, the action to which the biological or pharmaceutical product has been exposed and the certificate of compliance generated for the biological or pharmaceutical product. In other embodiments, the traceability record may further include some or all of the following information: the action to which the product has been subjected; physical conditions to which the biological or pharmaceutical product has been subjected; place and time where the biological or pharmaceutical product, has been used; person or machine responsible for performing said action; a password or permit for said action; and/or the control specifications to be fulfilled by the biological or pharmaceutical product. The traceability record can be stored in a remote database or alternatively can be stored, at least in part, in a memory of the radio frequency reader.

According to the invention, the given data of the certificate of compliance may further include at least one of the following information: place, time and date where the certificate of compliance has been generated; person or machine responsible for performing the action; the control specifications to be fulfilled by the biological or pharmaceutical product; a password or permit for the action; and/or redundant bits to check consistency of the certificate of compliance during the transmission, encryption keys or other security measures.

In case the certificate of compliance received by the radiofrequency sensor is not a first certificate of compliance, the radio frequency reader may overwrite a previous certificate of compliance stored in the memory of the radio frequency sensor with the recently received certificate of compliance.

In an embodiment, upon the confirmation that the radio frequency sensor data fulfills the given condition and/or is allowed for said given action is made, the radio frequency reader further sends a notification message to the radio frequency sensor or to an operator/user handling the biological or pharmaceutical product, for example via a software application (i.e. an APP) installed in a computer device operated by the operator/user or via a text message. The notification may include an audible, visual or electromagnetic indication.

In an embodiment, the transmitting of the generated certificate of compliance also includes the transmission of an audible, visual or electromagnetic message/indication.

In an embodiment, if the traceability record indicates that an event to which the biological or pharmaceutical product has been exposed is not according with the control specifications of the biological or pharmaceutical product a warning signal, either audible and/or visual, is generated.

Embodiments of the present invention also provide according to another aspect, a system for monitoring and preventing errors in the use of biological and pharmaceutical products. The proposed system includes a radio frequency sensor attached to a biological or pharmaceutical product to be used, the radio frequency sensor having associated thereto a unique identifier and including information about the biological or pharmaceutical product; a plurality of computing nodes participating in a certification system based on a blockchain protocol; and a radio frequency reader configured to wirelessly interact with the radio frequency sensor and with the plurality of computing nodes.

Particularly, according to the proposed system, the radio frequency reader, for example an RFID reader, is configured to:
- read, at least at a given period of time during said use, the unique identifier of the radio frequency sensor and information about the biological or pharmaceutical product, providing radio frequency sensor data;
- check whether the provided radio frequency sensor data fulfills a given condition and/or is allowed for a given action by comparing the radio frequency sensor data with control specifications of the biological or pharmaceutical product, the radio frequency reader being further configured to confirm that the radio frequency sensor data fulfills said given condition and/or is allowed for said given action;

- generate a certificate of compliance that validates that the biological or pharmaceutical product is compliant with said given condition and/or action, the certificate of compliance including a chain of bits coding a given data, the given data at least including an identifier of the certificate of compliance, the unique identifier of the radio frequency sensor and control specifications of the certificate of compliance;
- transmit the generated certificate of compliance to the radio frequency sensor and to the cited plurality of computing nodes participating in a certification system based on a blockchain protocol, the radio frequency sensor and each of the plurality of computing nodes further storing the certificate of compliance in a memory thereof; and
- generate a traceability record that indicates all the events to which the biological or pharmaceutical product has been exposed during said use, in view of the readings of the radio frequency sensor by the radio frequency reader.

In accordance with an embodiment, the system also includes a first database to store the control specifications of the biological or pharmaceutical product. Moreover, the system may also include a second database to store the generated traceability record.

In the above case, the first database and/or second database can be stored in a computing device, for example a PC, a tablet, a smartphone, a wearable device, etc. the computing device may have installed therein a cell phone or web-based application (e.g. an APP) for providing a user interface to operate the radio frequency reader, to access the information about the biological or pharmaceutical product included in the radio frequency sensor, to check the generated certificate of compliance and/or to receive a warning signal, either visual and/or audible.

Alternatively, the control specifications and/or the traceability record may be stored, at least in part, in a memory of the radio frequency reader.

### Brief Description of the Drawings

The previous and other advantages and features will be more fully understood from the following detailed description of embodiments, with reference to the attached figures, which must be considered in an illustrative and non-limiting manner, in which:
Fig. 1 is schematically illustrates the different elements that may form part of the system of the present invention.
Fig. 2 is a flow chart illustrating the steps of a method for monitoring and preventing errors in the use of biological and pharmaceutical products, according to an embodiment.

### Detailed Description of Preferred Embodiments

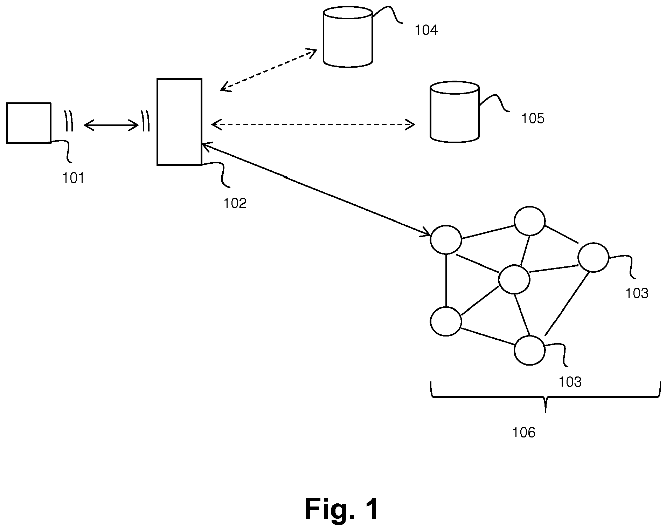

Fig. 1 shows a schematic illustration of a system of an example embodiment. According to this embodiment, the system includes, a radio frequency sensor 101, particularly a RFID sensor, which is attached to a biological or pharmaceutical product (not shown) to be used; a radio frequency reader 102, particularly a RFID reader; nodes 103 (e.g. computing machines) participating in a blockchain network 106 forming a certification system; a first database 104; and a second database 105.

Although not illustrated in Fig. 1, the system may comprise several radio frequency sensors 101, each one being attached to a different biological or pharmaceutical product. Moreover, the system may also comprise more than one radio frequency reader 102. A radio frequency reader 102 can be configured to wirelessly interact (i.e. read and/or write) with more than one radio frequency sensor 101.

It should be noted that a biological or pharmaceutical product, hereinafter referred as product, can be composed of several elements that are treated as a single unit (concept of lot). Moreover, a new product can be created as the result of the transformation or manipulation of the original product (concept of traceable antecessor or predecessor) and the information about the original product must remain known. The information about the product is an indispensable part of it so it must not be used in its absence, i.e. information about the donor, lab tests performed on the product or other.

The radio frequency sensor 101 particularly includes a memory, has a unique identifier stored in said memory, and stores information about the product. For example, the information about the product can include an identification of the product, information regarding its use safely, actions allowed and/or conditions to fulfill, a copy of a last certificate issued, among others. Optionally, the radio frequency sensor 101 can have a battery and processing means to execute operations such as arithmetical, logical, control, input/output operations, etc. A radio frequency sensor 101 can be also connected to or integrated in another radio frequency sensor 101, for example to measure physical parameters like temperature, pressure, irradiation, among others.

The radio frequency reader 102 is configured to read the unique identifier and the information of the radio frequency sensor 101 at different periods of time during the use of the product, providing radio frequency sensor data. The communication/interaction between the radio frequency reader 102 and the radio frequency sensor 101 can be made via radio frequency waves, for example. The radio frequency reader 102 can be also connected to a communication network, such as the Internet (not shown). Particularly, the radio frequency reader 102 has a memory and processing means (e.g. a central processing unit) with capacity to process arithmetical, logical, control, and/or input/output radio operations.

The database 104 (or first database as termed in the claims) is used to store control specifications of the product, for example the protocols, contracts or conditions to fulfil by the product and/or a list of actions allowed or denied for the product. The database 104 may also store data regarding the list of products identified by the radio frequency sensor 101, essential information of the product, physical conditions allowed for the product, the list of antecessors or predecessors of the product, if any, and/or other information considered useful. It should be noted that all or some of this data can be alternatively or complementary stored in the radio frequency sensor 101 or in the radio frequency reader 102, depending on its particular specifications/technology.

The database 105 (or second database as termed in the claims) is used to store a traceability record that indicates all the events to which the product has been exposed during its use. The traceability record mainly includes the unique identifier of the radio frequency sensor 101, the action to which the product has been exposed and a generated certificate of compliance. In other embodiments, the traceability record may further include the action to which the product has been subjected, physical conditions to which the product has been subjected, place and time where the product has been used, person or machine responsible for performing said action, a password or permit for said action and/or the control specifications to be fulfilled by the product. It should be noted that, alternatively or complementary, the traceability record can be stored in the memory of the radio frequency reader, depending on its particular specifications/technology. That is, the traceability record is a register of past event occurring to a product. Everything happened to a product is registered. So, it registers actions done on the product and it may register the actions tried to be done but denied, with all the information related to this event. It may register information requested by the database 104 and other convenient information.

Optionally, the system may also include a computing device (not shown in Fig. 1), for example a mobile phone, a wearable, a tablet, etc. having installed therein a software application. The computing device can store the database 104 and/or the database 105. That is, it can work as a prime repository or as a backup repository. The software application provides a user interface to communicate and send commands to the radio frequency reader 101, to access the information stored in the radio frequency sensor 101 and/or radio frequency reader 102, to receive alerts, etc.

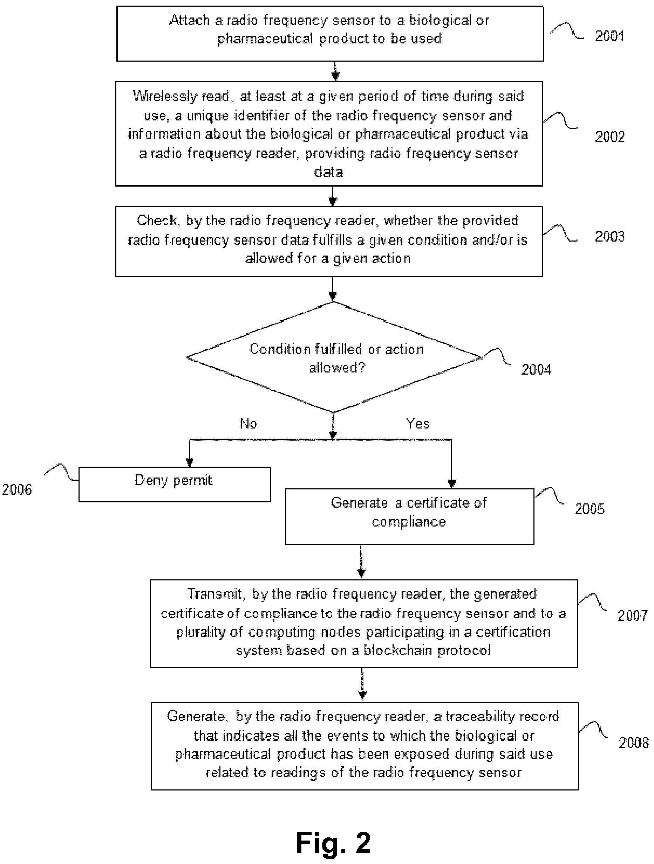

With reference now to Fig. 2, therein it is illustrated an embodiment of the proposed method. According to this embodiment, at step 2001, a radio frequency sensor 101 is attached to a product to be used. At step 2002, the radio frequency reader 101 wirelessly reads, at least at a given time during said use, the unique identifier and the information of the product stored in the radio frequency sensor 101, providing radio frequency sensor data. Then, at step 2003, the radio frequency reader 101 checks whether the radio frequency sensor data fulfills a given condition and/or is allowed for a given action. This is preferably done by comparing the radio frequency sensor data with the control specifications of the product. In case said given condition and/or action is allowed the radio frequency reader 102 generates, step 2005, a certificate of compliance that validates that the product is compliant with said given condition and/or action. On the contrary, step 2006, if said condition and/or action is not allowed, the radio frequency reader 102 denies the permit. The radio frequency reader 102 can send a notification message to the radio frequency sensor 101 and/or to the operator/user handling the product indicating the result of the cited checking.

At step 2007, after generation of the certificate of compliance, the radio frequency reader 102 transmits the generated certificate of compliance to the radio frequency sensor 101 and to the nodes 103. The radio frequency sensor 101 and each of the nodes 103 preferably store the received certificate of compliance in a memory thereof. Likewise, the radio frequency reader 102 can send a notification message to the radio frequency sensor 101 and to the nodes 103 to indicate that said transmission of the certificate of compliance has been made. Finally, at step 2008, the radio frequency reader 102 generates a traceability record that indicates all the events to which the product has been exposed during the use related to readings of the radio frequency sensor 101 (i.e. each action, and optionally each try or retry, allowed or not). In an embodiment, a warning signal (e.g. an alarm, audible and/or visual) is also generated if said traceability record indicates that an event to which the product has been exposed is not in accordance with its the control specifications.

The method can be configured to work either in offline (i.e. radio frequency reader not connected to a communication network) or online mode (radio frequency reader connected to a communication network). In offline, the tasks executed by the radio frequency reader 101 will be done as soon as it has access to the communication network.

The certificate of compliance particularly includes a chain of bits coding a given data forming a block. A certificate of compliance must at least include an identifier of the certificate of compliance, the unique identifier of the radio frequency sensor 101 and control specifications of the certificate of compliance. In addition, the certificate of compliance can also include the place, time and date where the certificate of compliance has been generated, the person or machine responsible for performing the action, the control specifications to be fulfilled by the product, a password or permit for the action and/or redundant bits to check consistency of the certificate of compliance during the transmission, encryption keys or other security measures. The different data coded in a block is particularly positioned sequentially, following a pattern previously established in a defined syntax, to make it possible to search, decode and/or understand.

In a particular embodiment, the certificate of compliance is generated by the radio frequency reader 102 in the following way. According to a contract, protocol or set of conditions of the product, the radio frequency reader 102 attempts to check a concrete specification so it wirelessly reads the data in the memory of the radio frequency sensor 101. The radio frequency reader 102 checks any existing certificate of compliance referred to the concrete set of control specifications to apply and then checks if the control specification is allowed of fulfilled for the radio frequency sensor 101 based on the data read, that may require a logical or arithmetical operation. If the control specification is allowed or fulfilled, the radio frequency reader 102 creates a new certificate of compliance placing the above detailed given data, in the form of a chain of bits forming a block, in a pre-determined order. The radio frequency reader 102 writes or overwrites the new certificate in the memory of the radio frequency sensor 101, in its own memory and it sends the certificate to the nodes 103. The radio frequency reader 102 may require a password or a permit prior to execute the action.

In an embodiment, the process of reading (or writing) in the memory of the radio frequency sensor 101 requires a security or authentication method (e.g. encryption, password, or any other).

Each of the nodes 103 works under common rules, independently from each other. Particularly, the nodes 103 follow some rules:
- The identity of the certificate issuer must be known with no possibility of counterfeit.
- The certificate cannot be modified by any mean. The nodes 103 must have credited procedures to avoid any change.
- The certificate must be able to be read by accredited third parties, following a procedure established.
- The system is more robust the more number of active nodes 103 the blockchain network 106 has. Optionally, the level of security of the blockchain network 106 can be established as a parameter depending on the number of nodes 103.
- Optionally, every node 103 has a list of the rest of nodes. There must be a process to update the list of active nodes.
- Optionally, the certificate has no expiration date although it may have an "end of validity" date. It persists while the certification system exists.
- Optionally, other convenient requirements.

According to a particular exemplary embodiment, a blood bag can only be transfused to a patient identified as ABC in the room 3 before 13:00 pm. All or part of this information of this particular product can be stored in a memory of its attached radio frequency sensor 101. Moreover, all or part of this product information can be also stored in a memory of the RFID reader 102. Other conditions can be given by other means (reading barcodes, sensors or other). The RFID reader 102 checks the ID of the RFID sensor 101 of the blood bag, a patient ID (e.g. a RFID wristband), a room ID (e.g. a barcode) and the time from its CPU clock. If all of them meet the condition, it is validated with a message. The RFID reader 102 then generates a certificate of compliance of all conditions, sends it to the nodes 103 and overwrites the last certificate in the radio frequency sensor 101 with the new one. Then, the RFID reader 10 generates a traceability record including, for example: the ID of the RFID sensor 101 of the blood bag, the RFID reader ID, a transfusion code, the patient ID, the room ID, the time and an ID of the certificate of compliance generated, (optionally) a medical staff ID, the flag of having sent the certificate of compliance to the nodes 103 and the acknowledge flag from the nodes 103.

In case any action is done after 13:00 pm, for example at 13.20 pm, the action will be denied. The RFID reader 102 will generate a traceability record where it stands that the RFID reader 102 has denied a transfusion of the blood bag ID to the patient ID in the room ID at 13:20pm by the medical staff ID.

Although various aspects of the invention are set out in the independent claims, other aspects of the invention comprise other combinations of features from the described embodiments and/or the dependent claims with the features of the independent claims, and not solely the combinations explicitly set out in the claims.

It is also noted herein that while the foregoing describes example embodiments of the invention, these descriptions should not be viewed in a limiting sense. Rather, there are several variations and modifications, which may be made without departing from the scope of the present invention.

The scope of the present invention is defined in the following set of claims.

## Claims

1. A method for monitoring and preventing errors in the use of biological and pharmaceutical products, the method comprising:
a) attaching a radio frequency sensor (101) to a biological or pharmaceutical product to be used, said radio frequency sensor (101) having associated thereto a unique identifier and including information about said biological or pharmaceutical product;
b) wirelessly reading, at least at a given period of time during said use, said unique identifier of the radio frequency sensor (101) and information about the biological or pharmaceutical product via a radio frequency reader (102), providing radio frequency sensor (101) data;
c) checking, by the radio frequency reader (102), whether the provided radio frequency sensor (101) data fulfills a given condition and/or is allowed for a given action by comparing the radio frequency sensor (101) data with control specifications of the biological or pharmaceutical product, the radio frequency reader (102) further confirming that the radio frequency sensor (101) data fulfills said given condition and/or is allowed for said given action;
d) generating, by the radio frequency reader (102), a certificate of compliance that validates that the biological or pharmaceutical product is compliant with said given condition and/or action, said certificate of compliance including a chain of bits coding a given data, said given data at least including an identifier of the certificate of compliance, the unique identifier of the radio frequency sensor (101) and control specifications of the certificate of compliance;
e) transmitting, by the radio frequency reader (102), the generated certificate of compliance to the radio frequency sensor (101) and to a plurality of computing nodes (103) participating in a certification system based on a blockchain protocol, the radio frequency sensor (101) and each of the plurality of computing nodes (103) further storing the certificate of compliance in a memory thereof; and
f) generating, by the radio frequency reader (102), a traceability record that indicates all the events to which the biological or pharmaceutical product has been exposed during said use in view of the readings of the radio frequency sensor (101) by the radio frequency reader (102).

2. The method of claim 1, wherein the control specifications of the biological or pharmaceutical product are stored in a first database (104) or at least in part, in a memory of the radio frequency reader (102), and include the protocols, contracts or conditions to fulfil by the biological or pharmaceutical product and/or a list of actions allowed or denied for the biological or pharmaceutical product.

3. The method of previous claims, wherein the traceability record is stored in a second database (105) or at least in part, in a memory of the radio frequency reader (102), and includes the unique identifier of the radio frequency sensor (101), the action to which the biological or pharmaceutical product has been exposed and the certificate of compliance generated for the biological or pharmaceutical product.

4. The method of claim 3, wherein the traceability record further includes at least some of the following information:
- the action to which the product has been subjected;
- physical conditions to which the biological or pharmaceutical product has been subjected;
- place and time where the biological or pharmaceutical product, has been used;
- person or machine responsible for performing said action;
- a password or permit for said action; and/or
- the control specifications to be fulfilled by the biological or pharmaceutical product.

5. The method of claim 1, wherein the certificate of compliance received by the radiofrequency sensor in said step e) is not a first certificate of compliance, the method further comprising overwriting a previous certificate of compliance stored in the memory of the radio frequency sensor (101) with the received certificate of compliance.

6. The method of claim 1, wherein in said step c) upon the confirmation that the radio frequency sensor (101) data fulfills the given condition and/or is allowed for said given action is made, the method further comprises sending, by the radio frequency reader (102), a notification message to the radio frequency sensor (101) or to a computed device operated by an operator/user handling the biological or pharmaceutical product, said notification message including an audible, visual or electromagnetic message.

7. The method of claim 1, wherein said transmitting of the generated certificate of compliance in step e) includes the transmission of an audible, visual or electromagnetic message.

8. The method of claim 1, further comprising generating a warning signal if said traceability record indicates that an event to which the biological or pharmaceutical product has been exposed is not according with the control specifications of the biological or pharmaceutical product.

9. The method of claim 1, wherein the given data of the certificate of compliance further including at least one of the following information:
- place, time and date where the certificate of compliance has been generated;
- person or machine responsible for performing the action;
- the control specifications to be fulfilled by the biological or pharmaceutical product;
- a password or permit for the action; and/or
- redundant bits to check consistency of the certificate of compliance during the transmission, encryption keys or other security measures.

10. A system for monitoring and preventing errors in the use of biological and pharmaceutical products, the system comprising:
- a radio frequency sensor (101) attached to a biological or pharmaceutical product to be used, said radio frequency sensor (101) having associated thereto a unique identifier and including information about said biological or pharmaceutical product;
- a plurality of computing nodes (103) participating in a certification system based on a blockchain protocol; and
- a radio frequency reader (102) configured to wirelessly interact with said radio frequency sensor (101) and with said plurality of computing nodes,
wherein the radio frequency reader (102) being configured to:
• read, at least at a given period of time during said use, said unique identifier of the radio frequency sensor (101) and information about the biological or pharmaceutical product, providing radio frequency sensor (101) data;
• check whether the provided radio frequency sensor (101) data fulfills a given condition and/or is allowed for a given action by comparing the radio frequency sensor (101) data with control specifications of the biological or pharmaceutical product, the radio frequency reader (102) being further configured to confirm that the radio frequency sensor (101) data fulfills said given condition and/or is allowed for said given action;
• generate a certificate of compliance that validates that the biological or pharmaceutical product is compliant with said given condition and/or action, said certificate of compliance including a chain of bits coding a given data, said given data at least including an identifier of the certificate of compliance, the unique identifier of the radio frequency sensor (101) and control specifications of the certificate of compliance;
• transmit the generated certificate of compliance to the radio frequency sensor (101) and to said plurality of computing nodes (103) participating in a certification system based on a blockchain protocol, the radio frequency sensor (101) and each of the plurality of computing nodes (103) further storing the certificate of compliance in a memory thereof; and
• generate a traceability record that indicates all the events to which the biological or pharmaceutical product has been exposed during said use, in view of the readings of the radio frequency sensor (101) by the radio frequency reader (102).

11. The system of claim 10, further comprising a first database (104) to store the control specifications of the biological or pharmaceutical product, wherein the control specifications include the protocols, contracts or conditions to fulfil by the biological or pharmaceutical product and/or a list of actions allowed or denied for the biological or pharmaceutical product.

12. The system of claim 10 or 11, further comprising a second database (105) to store the traceability record, wherein the traceability record includes the unique identifier of the radio frequency sensor (101), the action to which the biological or pharmaceutical product has been exposed and the certificate of compliance generated for the biological or pharmaceutical product.

13. The system of claim 10, wherein the radio frequency reader (102) is configured to store at least a part of the control specifications of the biological or pharmaceutical product and/or the traceability record in a memory thereof, wherein the control specifications include the protocols, contracts or conditions to fulfil by the biological or pharmaceutical product and/or a list of actions allowed or denied for the biological or pharmaceutical product, and wherein the traceability record includes the unique identifier of the radio frequency sensor (101), the action to which the biological or pharmaceutical product has been exposed and the certificate of compliance generated for the biological or pharmaceutical product.

14. The system of claim 11 or 12, wherein the first database (104) and/or second database (105) being stored in a computing device, the latter having installed therein a cell phone or web-based application for providing a user interface to operate the radio frequency reader (102), to access said information about said biological or pharmaceutical product included in the radio frequency sensor (101), to check the generated certificate of compliance and/or to receive a warning signal.
